(19) Europäisches Patentamt
European Patent Office
Office européen des brevets

(11) Publication number : 0 297 610 B1

# (12) EUROPEAN PATENT SPECIFICATION

(45) Date of publication of patent specification : 13.03.91 Bulletin 91/11

(51) Int. Cl.$^5$ : C07C 275/24, C07C 335/16, C07C 271/38

(21) Application number : 88110575.3

(22) Date of filing : 01.07.88

## (54) N-[(2,6-disubstituted)phenyl]-urea and carbamate inhibitors of acyl-CoA:cholesterol acyl-transferase.

The file contains technical information submitted after the application was filed and not included in this specification

(30) Priority : 02.07.87 US 69066

(43) Date of publication of application : 04.01.89 Bulletin 89/01

(45) Publication of the grant of the patent : 13.03.91 Bulletin 91/11

(84) Designated Contracting States : AT BE CH DE ES FR GB GR IT LI LU NL SE

(56) References cited :
EP-A- 0 007 184
EP-A- 0 257 888
AT-B- 191 903
DE-B- 1 102 722
DE-B- 1 246 722

(56) References cited :
DE-B- 1 643 848
DE-B- 2 716 540
GB-A- 1 112 760
US-A- 4 473 579

(73) Proprietor : WARNER-LAMBERT COMPANY
201 Tabor Road
Morris Plains New Jersey 07950 (US)

(72) Inventor : Roark, William Howard
1704 Cherokee
Ann Arbor Michigan 48104 (US)
Inventor : Roth, Bruce David
1440 King George Boulevard
Ann Arbor Michigan 48104 (US)
Inventor : Trivedi, Bharat Kalidas
44833 Tillotson Drive
Canton Michigan 48187 (US)

(74) Representative : Mansmann, Ivo
c/o Gödecke AG - Patentabteilung Postfach
569 Mooswaldallee 1-9
W-7800 Freiburg (DE)

EP 0 297 610 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to chemical compounds having pharmacological activity, to pharmaceutical compositions which include these compounds, and to a process for manufacturing such pharmaceutical compositions. More particularly, this invention concerns certain substituted urea and carbamate compounds which inhibit the enzyme acyl-coenzyme A : cholesterol acyltransferase (ACAT), pharmaceutical compositions containing these compounds, and a process for manufacturing pharmaceutical compositions useful in inhibiting intestinal absorption of cholesterol or regulating cholesterol.

In recent years the role which elevated blood plasma levels of cholesterol plays in pathological conditions in man has received much attention. Deposits of cholesterol in the vascular system have been indicated as causative of a variety of pathological conditions including coronary heart disease.

Initially, studies of this problem were directed toward finding therapeutic agents which would be effective in lowering total serum cholesterol levels. It is now known that cholesterol is transported in the blood in the form of complex particles consisting of a core of cholesteryl esters plus triglycerides and an exterior consisting primarily of phospholipids and a variety of types of protein which are recognized by specific receptors. For example, cholesterol is carried to the sites of deposit in blood vessels in the form of low density lipoprotein cholesterol (LDL cholesterol) and away from such sites of deposit by high density lipoprotein cholesterol (HDL cholesterol).

Following these discoveries, the search for therapeutic agents which control serum cholesterol turned to finding compounds which are more selective in their action ; that is, agents which are effective in elevating the blood serum levels of HDL cholesterol and/or lowering the levels of LDL cholesterol. While such agents are effective in moderating the levels of serum cholesterol, they have little or no effect on controlling the initial absorption of dietary cholesterol in the body through the intestinal wall.

In intestinal mucosal cells, dietary cholesterol is absorbed as free cholesterol which must be esterified by the action of the enzyme acyl-CoA : cholesterol acyltransferase (ACAT) before it can be packaged into the chylomicrons which are then released into the blood stream. Thus, therapeutic agents which effectively inhibit the action of ACAT prevent the intestinal absorption of dietary cholesterol into the blood stream or the reabsorption of cholesterol which has been previously released into the intestine through the body's own regulatory action.

The present invention provides a class of compounds with ACAT inhibitory activity having the structure of formula I

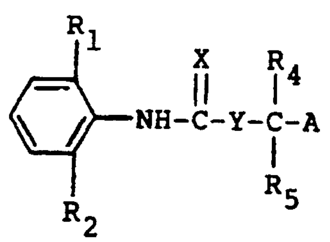

(I)

wherein $R_1$ and $R_2$ are independently selected from alkyl of from one to six carbon atoms ; fluorine ; chlorine ; bromine ; $-OR_3$ where $R_3$ is alkyl of from one to six carbon atoms, or $-CH_2$-phenyl.

The atom X is oxygen or sulfur, and Y is oxygen or –NH–.

The substituent groups $R_4$ and $R_5$ are independently hydrogen ; alkyl of from one to six carbon atoms ; or, taken together with the carbon atom to which they are attached, form a saturated carbocyclic ring of from three to eight carbon atoms, optionally substituted with alkyl of from one to four carbon atoms.

The group A is a straight or branched hydrocarbon chain of from six to seventeen carbon atoms optionally containing one or two double bonds.

The compounds of the present invention form a class of substituted ureas, thioureas, carbamates, and thiocarbamates having potent activity as inhibitors of the enzyme acyl CoA : cholesterol acyltransferase (ACAT). Preferred compounds of the present invention are the urea and thiourea compounds.

In the urea compounds of the present invention, the first nitrogen atom is monosubstituted by a (2,6-disubstituted)phenyl ring, in which the substituents are selected from alkyl of from one to six carbon atoms ; fluorine; chlorine ; bromine ; or –OR3 where $R_3$ is alkyl of from one to six carbon atoms, phenylmethyl, or phenylethyl. It has been found, in accordance with this invention that compounds in which the substitution pattern is 2,6-

disubstitution, are more potent inhibitors of ACAT than when the phenyl ring is monosubstituted, or disubstituted with a different substitution pattern.

The carbon atom immediately adjacent to A and to the nitrogen atom of the urea or thiourea compounds or immediately adjacent to A and to the oxygen atom of the carbamate or thiocarbamate compounds may be unsubstituted (i.e., bear two hydrogen atoms), monosubstituted (i.e., bear one hydrogen atom) or may be disubstituted by two groups $R_4$ and $R_5$ which may be the same or different, selected from alkyl of from one to six carbon atoms.

Alternatively, the groups $R_4$ and $R_5$ may form a saturated carbocyclic ring, together with the carbon atom to which they are attached, of from three to eight carbon atoms. US 4 473 579 describes compounds which are very potent inhibitors of the ACAT enzyme. These compounds however are N-tetrasubstituted urea and thiourea compounds which do not show the typical 2,6-substitution pattern of formula I in the aniline moiety. The compounds, therefore, are less active than the compounds of the present invention.

Examples of compounds contemplated as falling within the scope of the invention are the following :

N-[2-Ethyl-6-(1-methylethyl)phenyl]-N'-tetradecylurea.
(Z)-N-[2-Ethyl-6-(1-methylethyl)phenyl]-N'-9-octadecenylurea.
(Z)-N-(2,6-Dimethylphenyl)-N'-9-octadecenylthiourea.
(Z)-N-(2,6-Dimethylphenyl)-N'-9-octadecenyl-urea.
(Z)-N-(2-Chloro-6-methylphenyl)-N'-9-octadecenylurea.
(Z)-N-(2,6-Dichlorophenyl)-N'-9-octadecenylurea.
N-(2-Chloro-6-methylphenyl)-N'-tetradecylurea.
N-(2,6-Diethylphenyl)-N'-tetradecylurea. (Z)-N-(2,6-Diethylphenyl)-N'-9-octadecenylurea.
N-(2-Chloro-6-methylphenyl)-N'-octadecylurea.
N-[2,6-bis(1-Methylethyl)phenyl]-N'-octadecylurea.
N-(2,6-Diethylphenyl)-N'-tetradecylthiourea.
N-[2,6-bis(1-Methylethyl)phenyl]-N'-tetradecylthiourea.
N-Decyl-N'-(2,6-diethylphenyl)urea.
N-[2,6-bis(1-Methylethyl)phenyl]-N'-decylurea.
N-Decyl-N'-[2-ethyl-6-(1-methylethyl)-phenyl]urea.
N-Decyl-N'-[2-methyl-6-(1-methylethyl)phenyl]urea.
N-Decyl-N'-[2-(1,1-dimethylethyl)-6-methylphenyl]urea.
N-(2,6-Diethylphenyl)-N'-(1,1-dimethylundecyl)urea.
N-[2,6-bis(1-Methylethyl)phenyl]-N'-(1,1-dimethylundecyl)urea.
N-(1-Decylcyclopentyl)-N'-(2,6-diethylphenyl)urea.
N-[2,6-bis(1-Methylethyl)phenyl]-N'-(1-decylcyclopentyl)urea.

By the term "alkyl" as used throughout this specification and the appended claims is meant a branched or unbranched hydrocarbon grouping derived from a saturated hydrocarbon by the removal of a single hydrogen atom. Examples of alkyl groups contemplated as falling within the scope of this invention include methyl, ethyl, propyl, 1-methylethyl, butyl, 1-methylpropyl, 2-methylpropyl, 1,1-dimethylethyl pentyl, hexyl, and the like.

By the term "alkoxy" is meant an alkyl group, as defined above, attached to the parent molecular moiety through an oxygen atom.

The term "saturated carbocyclic ring" encompasses cyclopropyl, cyclobutyl, cyclopentyl, cyclohexyl, cycloheptyl, and cyclooctyl, as well as lower alkyl substituted carbocyclic rings such as methylcyclopropyl, methyl- or ethylcyclobutyl, etc.

The compounds of this invention may exist in unsolvated as well as solvated forms with pharmaceutically acceptable solvents such as water, ethanol and the like. In general, the solvated forms are considered equivalent to the unsolvated forms for the purposes of this invention.

The compounds of the present invention are prepared by the general method outline in Reaction Scheme 1. The appropriately substituted isocyanate, 2a (where X = O), or thioisocyanate compounds, 2b (where X = S), are reacted with the desired amine, 3, to obtain the substituted urea compounds of the present invention, 1a-1b, or with the desired alcohol, 4, to obtain the substituted carbamate compounds of the present invention, 1c-1d.

The reaction is generally carried out in a polar aprotic organic solvent such as ethyl acetate, at any temperature between room temperature and the boiling point of the solvent, with room temperature being preferred.

The reaction is allowed to proceed until analysis of the mixture by a means such as chromatography indicates that the reaction is substantially complete. Reaction times may vary between about two hours to about 24 hours, depending upon the particular reagents and reaction temperature employed. The starting isocyanate, thioisocyanate, amine, and alcohol compounds for making the compounds of this invention are known or commercially available or, if not previously known, are prepared by methods well known in the art.

The urea compounds of the present invention where the substituent $R_4$ and $R_5$ form a saturated ring as

defined are prepared according to the method described in Scheme 2. Reaction of cyclic carboxylic acid 5 with LDA and appropriate electrophile Br-A at low temperature gave the acid compound 6 which was converted to isocyananate derivative (7) in a standard manner. The isocyananate compound 7 was treated with an appropriately substituted amine compound (8) to yield the title urea compound 1.

## Reaction Scheme 1

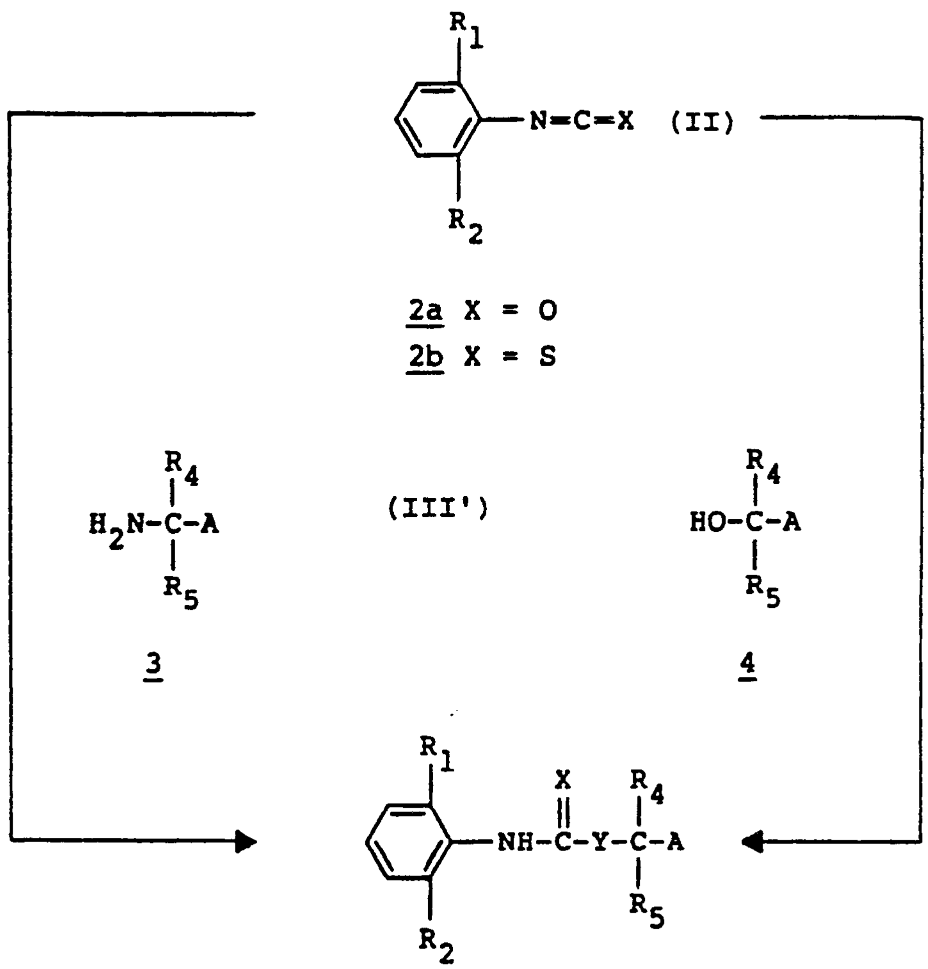

2a X = O
2b X = S

(III')

3

4

1a X = O, Y = NH
1b X = S, Y = NH
1c X = O, Y = O
1d X = S, Y = O

Reaction Scheme 2

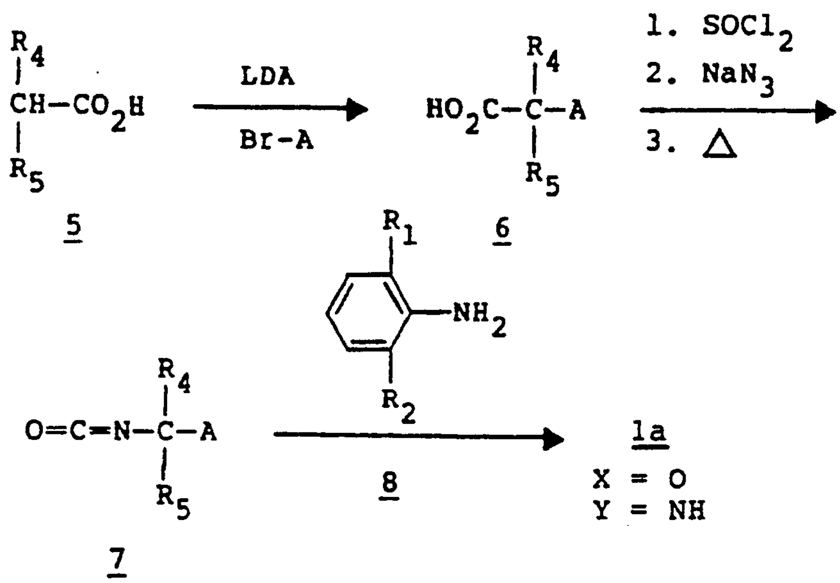


As shown by the data presented below in Table 1, the compounds of the present invention are potent inhibitors of the enzyme acyl-CoA : cholesterol acyltransferase (ACAT), and are thus effective in inhibiting the esterification and transport of cholesterol across the intestinal cell wall. The compounds of the present invention are thus useful in pharmaceutical formulations for the inhibition of intestinal absorption of dietary cholesterol or the reabsorption of cholesterol released into the intestine by normal body action.

In Vitro Tests

The ability of representative compounds of the present invention to inhibit ACAT was measured using an in vitro test more fully described in Field, F. J. and Salone, R. G., Biochemica et Biophysica 712 : 557-570 (1982). The test assesses the ability of a test compound to inhibit the acylation of cholesterol by oleic acid by measuring the amount of radiolabeled cholesterol oleate formed from radiolabeled oleic acid in a tissue preparation containing rabbit intestinal microsomes.

The data appear in Table 1 where they are expressed as $IC_{50}$ values ; i.e. the concentration of test compound required to inhibit 50% expression of the enzyme.

Table 1

| Compound of Example | $IC_{50}$ (Micromolar) |
|---|---|
| 1 | 0.019 |
| 2 | 0.037 |
| 3 | 10.0 |
| 4 | 0.16 |
| 5 | 0.18 |
| 6 | 0.4 |
| 7 | 0.027 |
| 8 | 0.021 |
| 9 | 0.043 |
| 10 | 1.1 |
| 11 | 0.048 |
| 12 | 0.42 |
| 13 | ----- |
| 14 | 0.1 |
| 15 | 0.056 |
| 16 | 0.087 |
| 17 | 0.21 |
| 18 | 0.20 |
| 19 | 0.052 |
| 20 | 0.045 |
| 21 | 0.10 |
| 22 | ----- |

In Vivo Tests

In an in vivo screen, designated PCC, male Sprague-Dawley rats (approximately 200 g body weight) are randomly divided into groups and provided ad libidum a regular chow diet (Purina No. 5002, Ralston Purina Co., 711 West Fuesser Road, Mascoutah, Illinois, 62224, USA), supplemented with 5.5% peanut oil, 1.5% choles-terol, and 0.3%-0.5% cholic acid, together with 0.05% of the test drug which is admixed into the diet. After one week the animals are etherized and a blood sample is taken from the heart and mixed with 0.14% ethylene-diamine tetraacetic acid (EDTA) to measure the total cholesterol. The results of this trial for representative compounds of the present invention appear in Table 2.

Table 2

| Compound of Example | Total Blood Cholesterol (mg/dl) | % Change |
|---|---|---|
| 19 | 73 (Control = 181) | -60 |
| 1 | 106 (Control = 181) | -41 |
| 2 | 107 (Control = 181) | -41 |

In therapeutic use as agents for the inhibition of intestinal absorption of cholesterol, or as hypolipidemic or hypocholesterolemic agents, the compounds utilized in the pharmaceutical method of this invention are administered to the patient at dosage levels of from 250 to 1000 mg per day. For a normal human adult of approximately 70 kg of body weight, this translates into a dosage of from 5 to 20 mg/kg of body weight per day. The specific dosages employed, however, may be varied depending upon the requirements of the patient, the severity of the condition being treated, and the activity of the compound being employed. The determination of optimum dosages for a particular situation is within the skill of the art.

For preparing pharmaceutical compositions from the compounds of this invention, inert, pharmaceutically acceptable carriers can be either solid or liquid. Solid form preparations include powders, tablets, dispersible granules, capsules, and cachets.

A solid carrier can be one or more substances which may also act as diluents, flavoring agents, solubilizers, lubricants, suspending agents, binders, or tablet disintegrating agents ; it can also be an encapsulating material.

In powders, the carrier is a finely divided solid which is in a mixture with the finely divided active component. In tablets, the active compound is mixed with the carrier having the necessary binding properties in suitable proportions and compacted in the shape and size desired.

Powders and tablets preferably contain between about 5 to about 70% by weight of the active ingredient. Suitable carriers are magnesium carbonate, magnesium stearate, talc, lactose, sugar, pectin, dextrin, starch, tragacanth, methyl cellulose, sodium carboxymethyl cellulose, a low-melting wax, cocoa butter, and the like.

The term "preparation" is intended to include the formulation of the active compound with encapsulating material as a carrier providing a capsule in which the active component (with or without other carriers) is surrounded by a carrier, which is thus in association with it. In a similar manner, cachets are also included.

Tablets, powders, cachets, and capsules can be used as solid dosage forms suitable for oral administration.

Liquid form preparations include solutions suitable for oral administration, or suspensions and emulsions suitable for oral administration. Aqueous solutions for oral administration can be prepared by dissolving the active compound in water and adding suitable flavorants, coloring agents, stabilizers, and thickening agents as desired. Aqueous suspensions for oral use can be made by dispersing the finely divided active component in water together with a viscous material such as natural or synthetic gums, resins, methyl cellulose, sodium carboxymethyl cellulose, and other suspending agents known to the pharmaceutical formulation art.

Preferably, the pharmaceutical preparation is in unit dosage form. In such form, the preparation is divided into unit doses containing appropriate quantities of the active component. The unit dosage form can be a packaged preparation, the package containing discrete quantities of the preparation, for example, packeted tablets, capsules, and powders in vials or ampoules. The unit dosage form can also be a capsule, cachet, or tablet

itself, or it can be the appropriate number of any of these packaged forms.

The following preparative examples are provided to enable one skilled in the art to practice the invention, and are illustrative thereof.

EXAMPLE 1

N-[2-Ethyl-6-(1-methylethyl)-phenyl]-N'-tetradecylurea

2-Ethyl-6-isopropyl-phenylisocyanate (1.89 g, 0.01 mole) was added to a solution of 1-tetradecylamine (2.13 g, 0.01 mole) in 60 ml ethyl acetate at room temperature. The flask was gently agitated and the product precipitated immediately. The reaction mixture was warmed to 50°C for a few minutes and then cooled to room temperature. The white solid was collected by filtration and air dried. In this way 3.40 g of product was obtained.

Analysis calculated for $C_{26}H_{46}N_2O$
C = 77.56 ; H = 11.51 ; N = 6.96
Found C = 78.12 ; H = 11.84 ; N = 7.09

EXAMPLE 2

(Z)-N-[2-Ethyl-6-(1-methylethyl)-phenyl]-N'-9-octadecenylurea

2-Ethyl-6-isopropyl-phenylisocyanate (1.89 g, 0.01 mole) was added to (3.16 g, 0.012 mole) technical oleylamine in 50 ml ethyl acetate at room temperature. The solution was heated to 50°C, then the solvent removed on the rotary evaporator. The residue was redissolved in ethyl acetate and washed with dilute aqueous HCl, aqueous NaCl solution, the layers separated and the organic layer dried over $MgSO_4$. The solution was filtered and concentrated yielding an oil, which was purified by flash chromatography on $SiO_2$ using 50/50 hexane/ethyl acetate as eluant. In this way 4.13 g of product was obtained as an oil.

Analysis calculated for $C_{30}H_{52}N_2O$
C = 78.89 ; H = 11.47 ; N = 6.13
Found C = 78.50 ; H = 11.74 ; N = 5.89

EXAMPLE 3

(Z)-N-(2,6-Dimethylphenyl)-N'-9-octadecenylthiourea

The title compound was prepared according to Example 2.

Analysis calculated for $C_{27}H_{46}N_2S$
C = 75.29 ; H = 10.76 ; N = 6.50
Found C = 75.17 ; H = 10.67 ; N = 6.22

EXAMPLE 4

(Z)-N-(2,6-Dimethylphenyl)-N'-9-octadecenylurea

The title compound was prepared according to Example 1.

C = 78.21 ; H = 11.18 ; N = 6.76
Found C = 78.58 ; H = 10.98 ; N = 6.89

EXAMPLE 5

(Z)-N-(2-Chloro-6-methylphenyl)-N'-9-octadecenylurea

The title compound was prepared according to Example 1.

Analysis calculated for $C_{26}H_{43}ClN_2O$
C = 71.77 ; H = 9.96 ; N = 6.44
Found C = 71.63 ; H = 9.55 ; N = 6.59

EXAMPLE 6

(Z)-N-(2,6-Dichlorophenyl)-N′-9-octadecenylurea

The title compound was prepared according to Example 1.
Analysis calculated for $C_{25}H_{40}Cl_2N_2O$
C = 65.92 ; H = 8.85 ; N = 6.15
Found C= 65.96 ; H = 9.14 ; N = 6.21

EXAMPLE 7

N-(2-Chloro-6-methylphenyl)-N′-tetradecylurea

The title compound was prepared according to Example 1.
Analysis calculated for $C_{22}H_{37}ClN_2O$
C = 69.35 ; H = 9.79 ; N = 7.35
Found C = 69.58 ; H = 9.89 ; N = 7.27

EXAMPLE 8

N-(2,6-Diethylphenyl)-N′-tetra-decylurea

The title compound was prepared according to Example 1.
Analysis calculated for $C_{25}H_{44}N_2O$
C = 77.26 ; H = 11.41 ; N = 7.21
Found C = 77.57 ; H = 11.51 ; N = 7.29

EXAMPLE 9

(Z)-N-(2,6-Diethylphenyl)-N′-9-octadecenylurea

The title compound was prepared according to Example 2.
Analysis calculated for $C_{29}H_{50}N_2O$
C = 78.68 ; H = 11.38 ; N = 6.33
Found C = 79.43 ; H = 11.78 ; N = 6.47

EXAMPLE 10

N-(2-Chloro-6-methylphenyl)-N′-octadecylurea

The title compound was prepared according to Example 1
Analysis calculated for $C_{26}H_{45}ClN_2O$
C = 71.44 ; H = 10.38 ; N = 6.41
Found C = 71.99 ; H = 10.65 ; N = 6.28

EXAMPLE 11

N-[2,6-bis(1-Methylethyl)phenyl]-N′-octadecylurea

The title compound was prepared according to Example 1.
Analysis calculated for $C_{31}H_{56}N_2O$
C = 78.75 ; H = 11.94 ; N = 5.93
Found C = 78.86 ; H = 12.13 ; N = 5.93

EXAMPLE 12

N-(2,6-Diethylphenyl)-N'-tetradecylthiourea

The title compound was prepared according to Example 1.
Analysis calculated for $C_{25}H_{44}N_2S$
C = 74.20 ; H = 10.96 ; N = 6.92
Found C = 74.32 ; H = 11.25 ; N = 6.96

EXAMPLE 13

N-[2,6-bis(1-methylethyl)phenyl]-N'-tetradecylthiourea

The title compound was prepared according to Example 1.
Analysis calculated for $C_{27}H_{48}N_2S$
C = 74.94 ; H = 11.18 ; N = 6.47
Found C = 75.00 ; H = 11.33 ; N = 6.45

EXAMPLE 14

N-Decyl-N'-(2,6-diethylphenyl)urea

The title compound was prepared according to Example 1.
Analysis calculated for $C_{21}H_{36}N_2O$
C = 75.85 ; H = 10.91 ; N = 8.42
Found C = 75.94 ; H = 10.91 ; N = 8.31

EXAMPLE 15

N-[2,6-bis(1-Methylethyl)phenyl]-N'-decylurea

The title compound was prepared according to Example 1.
Analysis calculated for $C_{23}H_{40}N_2O$
C = 76.61 ; H = 11.18 ; N = 7.77
Found C = 76.32 ; H = 11.25 ; N = 7.56

EXAMPLE 16

N-Decyl-N'-[2-ethyl-6-(1-methyl-ethyl)phenyl]urea

The title compound was prepared according to Example 1.
Analysis calculated for $C_{22}H_{38}N_2O$
C = 76.25 ; H = 11.05 ; N = 8.08
Found C = 76.38 ; H = 11.13 ; N = 8.01

EXAMPLE 17

N-Decyl-N'-[2-methyl-6-(l-methylethyl)-phenyl]urea.

The title compound was prepared according to Example 1.
Analysis calculated for $C_{21}H_{36}N_2O$
C = 75.85 ; H = 10.91 ; N = 8.42
Found C = 75.95 ; H = 11.15 ; N = 8.31

EXAMPLE 18

N-Decyl-N'-[2-(1,1-dimethylethyl)-6-methylphenyl]urea

The title compound was prepared according to Example 1.

Analysis calculated for $C_{22}H_{38}N_2O$

C = 76.25 ; H = 11.05 ; N = 8.08

Found C = 76.31 ; H = 11.30 ; N = 7.90

EXAMPLE 19

N-(2,6-Diethylphenyl)-N'-(1.1-dimethylundecyl)urea

Step A : Preparation of 2-isocyanato-2-methyl dodecane.

2,2-Dimethyl dodecanoic acid (99.6 g, 0.44 M) was dissolved in 500 ml of thionyl chloride. The mixture was stirred at room temperature for 24 hours. The mixture was then heated at reflux for two hours. The cooled reaction mixture was concentrated in vacuo. Diethyl ether was added to the residue and this solution was concentrated in vacuo. The resulting oil was distilled to yield 97.0 g, (BP 85-87° at 0.15 mm) of 2,2-dimethyl dodecanoyl chloride.

2,2-dimethyl dodecanoyl chloride (73.95 g, 0.3 M) was dissolved in 100 ml dry acetone. This solution was added dropwise with vigorous stirring to a solution of sodium azide (29.25 g, 0.45 M) dissolved in 100 ml water. The temperature was maintained between 13° and 16°C by use of an ice-bath. The reaction mixture was stirred for 20 minutes. The layers were separated and the aqueous layer washed once with hexane. The hexane solution was combined with the organic layer. The combined organic solution was washed once with a saturated brine solution, dried over $MgSO_4$, and concentrated in vacuo to yield 75.9 g of a clear oil. This oil was taken up in 100 ml benzene. The resulting solution was added dropwise, with vigorous stirring, to 500 ml of benzene heated to 60°. Stir at 70° for an additional two hours. The reaction mixture was concentrated in vacuo and the product distilled to yield 58.0 g, (BP 77-80 at 0.05 mm) of 2-isocyanate-2-methyl dodecane.

Step B : Preparation of N-(2,6-diethylphenyl)-N'-(1,1-dimethyl undecyl)urea.

2-Isocyanato-2-methyl dodecane (2.25 g, 0.01 M) and 2,6-diethyl aniline (1.5 g, 0.01 M) were mixed together and heated on a steam bath for 16 hours. The cooled reaction mixture was taken up in ether. The ether solution was washed with 2N HCl ; saturated sodium bicarbonate, brine, and dried over $MgSO_4$. The ether solution was concentrated in vacuo to yield 3.2 g of the crude compound. This was purified by chromatography on silica, eluting with 80% hexane : 20% ethyl acetate (volume/volume) to yield 1.4 g of the title compound as an oil that quickly solidified, mp 60-62°.

Analysis for $C_{24}H_{42}N_2O$

C = 76.95 ; H = 11.30 ; N = 7.48

Found C = 76.87 ; H = 11.49 ; N = 7.38

EXAMPLE 20

N-[2.6-bis(1-Methylethyl)phenyl]-N'-(1,1-dimethylundecyl)urea

Using the general method of Example 19, but starting with 4.5 g (0.02 M) of 2-isocyanato-2-methyl dodecane and 3.54 g (0.02 M) 2,6-diisopropyl aniline there was prepared 2.3 g of an oil which was purified by chromatography on silica, eluting with 80% hexane :20% ethyl acetate (volume/volume).

Analysis for $C_{26}H_{48}N_2O$

C = 77.55 ; H = 11.52 ; N = 6.96

Found C = 77.92 ; H = 11.79 ; N = 6.67

EXAMPLE 21

N-(1-Decylcyclopentyl)-N'-(2.6-diethylphenyl)urea

Using the general method of Example 19 but starting with 92.3 g (0.32 mm) 1-decyl cyclopentane carboxylic

acid and 300 ml of thionyl chloride. There was prepared 74.8 g of 1-decylcyclopentane carboxyl chloride.

Starting with 12.7 g (0.046 M) of 1-decyl cyclopentane carboxyl chloride and 4.9 g (0.075 M) sodium azide. There was prepared 12.2 g of 1-decyl-1-isocyanotocyclopentane.

Starting with 6.1 g (0.024 M) of 1-decyl-1-isocyanatocyclopentane and 3.58 g (0.024 M) 2,6-diethyl aniline, there was prepared 4.5 g of N-(1-decylcyclopentyl)-N'-(2,6-diethylphenyl)urea, mp 82-85° recrystallized from ethyl acetate.

Analysis for $C_{26}H_{44}N_2O$

C = 77.94 ; H = 11.07 ; N = 6.99

Found C = 77.80 ; H = 11.23 ; N = 6.73

EXAMPLE 22

N-[2-6-bis(1-Methylethyl)-pheny1]-N'-(1-decylcyclopentyl)urea

Using the general method of Example 19, but starting with 5.9 g (0.024 M) of 1-decyl-1-isocyanatocyclopentane and 4.2 g (0.024 M) of 2,6-diisopropylaniline, there was prepared 2.3 g of N-[2,6-bis(1-methylethyl)phenyl]-N'-(1-decylcyclopentyl)urea, after purification by chromatography on silica, eluting with 80% hexane : 20% ethyl acetate (volume/volume), mp 105-107°C.

Analysis for $C_{28}H_{48}N_2O$

C = 78.44 ; H = 11.29 ; N = 6.54

Found C = 78.47 ; H = 11.43 ; N = 6.48

## Claims

### Claims for the Contracting States AT, BE, CH, DE, FR, GB, IT, LI, LU, NE, SE

1. A compound having the formula I

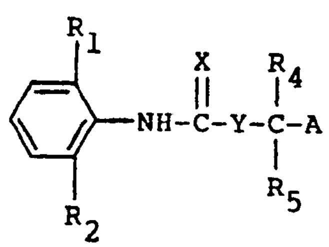

(I),

wherein

$R_1$ and $R_2$ are independently selected from alkyl of from one to six carbon atoms ;
fluorine ;
chlorine ;
bromine ;
$OR_3$ where $R_3$ is selected from alkyl of from one to six carbon atoms ;
or phenylmethyl ;

X is oxygen or sulfur ;

Y is oxygen or –NH– ;

$R_4$ and $R_5$ are independently selected from hydrogen ;
alkyl of from one to six carbon atoms ; or when taken together with the carbon atom to which they are attached, $R_4$ and $R_5$ form a saturated carbocyclic ring of from three to eight carbon atoms optionally substituted with alkyl of from one to four carbon atoms ; and

A is straight or branched alkyl of from six to seventeen carbon atoms, optionally containing one or two carbon-carbon double bonds.

2. A compound as defined by Claim 1 wherein X is oxygen.

3. A compound as defined by Claim 1 wherein X is sulfur.

4. A compound as defined by Claim 1 wherein Y is –NH–

5. A compound as defined by Claim 1 selected from the group consisting of
N-[2-ethyl-6-(1-methylethyl)phenyl]-N'-tetradecyl-urea,
(Z)-N-[2-ethyl-6-(1-methylethyl)phenyl]-N'-9-octadecenylurea,
(Z)-N-(2,6-dimethylphenyl)-N'-9-octa-decenylthiourea,
(Z)-N-(2,6-dimethylphenyl)-N'-9-octa-decenylurea,
(Z)-N-(2-chloro-6-methylphenyl)-N'-9-octadecenylurea,
(Z)-N-(2,6-dichlorophenyl)-N'-9-octa-decenylurea,
N-(2-chloro-6-methylphenyl)-N'-tetra-decylurea,
N-(2,6-diethylphenyl)-N'-tetradecylurea,
(Z)-N-(2,6-diethylphenyl)-N'-9-octa-decenylurea,
N-(2-chloro-6-methylphenyl)-N'-octa-decylurea,
N-[2,6-bis(1-methylethyl)phenyl]-N'-octadecylurea,
N-(2,6-diethylphenyl)-N'-tetradecylthiourea,
N-(2,6-bis(1-methylethyl)phenyl]-N'-tetradecylthiourea,
N-decyl-N'-(2,6-diethylphenyl)urea,
N-[2,6-bis(1-methylethyl)phenyl]-N'-decylurea,
N-decyl-N'-[2-methyl-6-(1-methylethyl)-phenyl]urea,
N-decyl-N'-[2-methyl-6-(1-methylethyl)-phenyl]urea,
N-decyl-N'-[2-(1,1-dimethylethyl)-6-methylphenyl]urea,
N-(2,6-diethylphenyl)-N'-(1,1-dimethyl-undecyl)urea,
N-[2,6-bis(1-methylethyl)phenyl]-N'-(1,1-dimethyl-undecyl)urea,
N-(1-decylcyclopentyl)-N'-(2,6-diethylphenyl)urea,
N-[2,6-bis(1-methylethyl)phenyl]-N'-(1-decylcyclopentyl)urea.

6. A pharmaceutical composition containing a compound as defined by Claim 1 in combination with a pharmaceutically acceptable carrier.

7. A process for manufacturing a pharmaceutical composition according to Claim 6 for regulating cholesterol.

8. A process of preparing a compound having the formula I comprising the steps of:

a) reacting a compound of the formula II

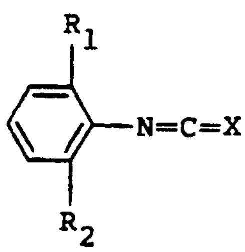

(II),

wherein $R_1$ and $R_2$ and X are as defined above with a compound having the formula III

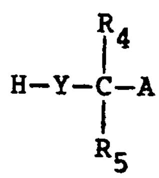

(III),

wherein Y, $R_4$, and $R_5$ are as defined above in a polar aprotic organic solvent at ambient temperature ; and

b) thereafter isolating and purifying the product of said reaction step.

**Claims for the Contracting States GR, ES**

1. A process of preparing a compound having the formula I

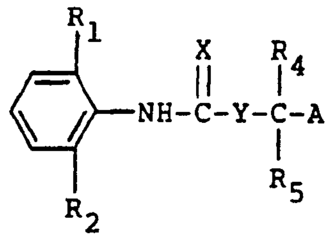

(I),

wherein

$R_1$ and $R_2$ are independently selected from alkyl of from one to six carbon atoms ;
fluorine ;
chlorine ;
bromine ;
$OR_3$ where $R_3$ is selected from alkyl of from one to six carbon atoms ;
or phenylmethyl ;

X is oxygen or sulfur ;

Y is oxygen or –NH– ;

$R_4$ and $R_5$ are independently selected from hydrogen ;
alkyl of from one to six carbon atoms ; or when taken together with the carbon atom to which they are attached, $R_4$ and $R_5$ form a saturated carbocyclic ring of from three to eight carbon atoms optionally substituted with alkyl of from one to four carbon atoms ; and

A is straight or branched alkyl of from six to seventeen carbon atoms, optionally containing one or two carbon-carbon double bonds, comprising the steps of :

a) reacting a compound of the formula II

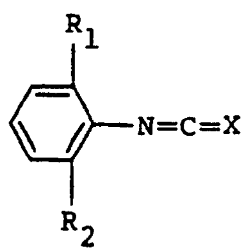

(II),

wherein $R_1$ and $R_2$ and X are as defined above with a compound having the formula III

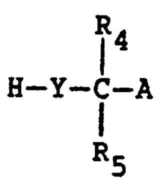

(III),

wherein Y, $R_4$, and $R_5$ are as defined above in a polar aprotic organic solvent at ambient temperature ; and

b) thereafter isolating and purifying the product of said reaction step.

2. A process of preparing a compound as defined by Claim 1 wherein X is oxygen.

3. A process of preparing a compound as defined by Claim 1 wherein X is sulfur.

4. A process of preparing a compound as defined by Claim 1 wherein Y is –NH–.

5. A process of preparing a compound as defined by Claim 1 selected from the group consisting of

N-[2-ethyl-6-(1-methylethyl)phenyl]-N'-tetradecyl-urea,
(Z)-N-[2-ethyl-6-(1-methylethyl)phenyl]-N'-9-octadecenylurea,
(Z)-N-(2,6-dimethylphenyl)-N'-9-octa-decenylthiourea,
(Z)-N-(2,6-dimethylphenyl)-N',-9-octa-decenylurea,
(Z)-N-(2-chloro-6-methylphenyl)-N'-9-octadecenylurea,
(Z)-N-(2,6-dichlorophenyl)-N'-9-octa-decenylurea,
N-(2-chloro-6-methylphenyl)-N'-tetra-decylurea,
N-(2,6-diethylphenyl)-N'-tetradecylurea,
(Z)-N-(2,6-diethylphenyl)-N'-9-octa-decenylurea,
N-(2-chloro-6-methylphenyl)-N'-octa-decylurea,
N-[2,6-bis(1-methylethyl)phenyl]-N'-octadecylurea,
N-(2,6-diethylphenyl)-N'-tetradecylthiourea,
N-[2,6-bis(1-methylethyl)phenyl]-N'-tetradecylthiourea,
N-decyl-N'-(2,6-diethylphenyl)urea,
N-[2,6-bis(1-methylethyl)phenyl]-N'-decylurea,
N-decyl-N'-[2-ethyl-6-(1-methylethyl)-phenyl]urea.
N-decyl-N'-[2-methyl-6-(1-methylethyl)-phenyl]urea,
N-decyl-N'-[2-(1,1-dimethylethyl)-6-methylphenyl]urea,
N-(2,6-diethylphenyl)-N'-(1,1-dimethyl-undecyl)urea,N-[2,6-bis(1-methylethyl)phenyl]-N'-(1,1-dimethyl-undecyl)urea,
N-(1-decylcyclopentyl)-N'-(2,6-diethylphenyl)urea,
N-[2,6-bis(1-methylethyl)phenyl]-N'-(1-decylcyclopentyl)urea.

6. A method of use of a compound prepared according to claim 1 for manufacturing a pharmaceutical composition for regulating cholesterol.

## Ansprüche

### Patentansprüche für die Vertragsstaaten AT, BE, CH, DE, FR, GB, IT, LI, LU, NE, SE

1. Verbindungen der Formel I

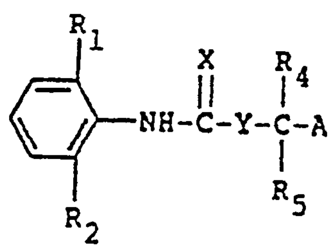

(I),

worin

$R_1$ und $R_2$, die unabhängig voneinander Alkyl mit 1 bis 6 Kohlenstoffatomen, Fluor, Chlor, Brom, $-OR_3$, worin $R_3$ Alkyl mit 1 bis 6 Kohlenstoffatomen oder $-CH_2$-phenyl ;

X Sauerstoff oder Schwefel ;

Y Sauerstoff oder –NH– ;

$R_4$ und $R_5$ unabhängig voneinander Wasserstoff, Alkyl mit 1 bis 6 Kohlenstoffatomen oder zusammen mit dem Kohlenstoffatom, an das sie gebunden sind, einen gesättigten carbocyclischen Ring mit 3 bis 8 Kohlenstoffatomen, der gegebenenfalls mit Alkyl mit 1 bis 4 Kohlenstoffatomen substituiert ist ; und

A eine geradkettige oder verzweigte Kohlenwasserstoffkette mit 7 bis 17 Kohlenstoffatomen, welche gegebenenfalls eine oder zwei Doppelbindungen enthält, bedeuten.

2. Verbindungen wie in Anspruch 1 definiert, worin X Sauerstoff bedeutet.

3. Verbindungen wie in Anspruch 1 definiert, worin X Schwefel bedeutet.

4. Verbindungen wie in Anspruch 1 definiert, worin Y –NH– bedeutet.

5. Verbindungen wie in Anspruch 1 definiert, ausgewählt aus der aus den nachfolgen aufgeführten Verbindungen bestehenden Gruppe

N-[2-Ethyl-6-(1-methylethyl)phenyl]-N'-tetradecylharnstoff ;

(Z)-N-[2-Ethyl-6-(1-methylethyl)phenyl]-N'-9-octadecenylharnstoff ;
(Z)-N-(2,6-Dimethylphenyl)-N'-9-octadecenylthioharnstoff ;
(Z)-N-(2,6-Dimethylphenyl)-N'-9-octadecenylharnstoff ;
(Z)-N-(2-Chlor-6-methylphenyl)-N'-9-octadecenylharnstoff ;
(Z)-N-(2,6-(Dichlorphenyl)-N'-9-octadecenylharnstoff ;
N-(2-Chlor-6-methylphenyl)-N'-tetradecylharnstoff ;
N-(2,6-Diethylphenyl)-N'-tetradecylharnstoff ;
(Z)-N-(2,6-Diethylphenyl)-N'-9-octadecenylharnstoff ;
N-(2-Chlor-6-methylphenyl)-N'-octadecylharnstoff ;
N-[2,6-bis(1-Methylethyl)phenyl]-N'-octadecylharnstoff ;
N-(2,6-Diethylphenyl)-N'-tetradecylthioharnstoff ;
N-[2,6-bis(1-Methylethyl)phenyl]-N'-tetradecylthioharnstoff ;
N-Decyl-N'-(2,6-diethylphenyl)-harnstoff ;
N-[2,6-bis(1-Methylethyl)phenyl]-N'-decylharnstoff ;
N-Decyl-N'-[2-ethyl-6-(1-methylethyl)phenyl]-harnstoff ;
N-Decyl-N'-[2-methyl-6-(1-methylethyl)phenyl]-harnstoff ;
N-Decyl-N'-[2-(1,1-dimethylethyl)-6-methylphenyl]-harnstoff ;
N-(2,6-Diethylphenyl)-N'-(1,1-dimethylundecyl)-harnstoff ;
N-[2,6-bis(1-Methylethyl)phenyl]-N'-(1,1-dimethylundecyl)harnstoff ;
N-(1-Decylcyclopentyl)-N'-(2,6-diethylphenyl)harnstoff ;
N-[2,6-bis(1-Methylethyl)phenyl]-N'-(1-decylcyclopentyl)harnstoff.

6. Pharmazeutische Zubereitungen, enthaltend eine Verbindung wie in Anspruch 1 definiert zusammen mit einem pharmazeutisch annehmbaren Trägerstoff.

7. Verfahren zur Herstellung einer pharmazeutischen Zubereitung nach Anspruch 6 zur Regulierung von Cholesterin.

8. Verfahren zur Herstellung einer Verbindung der Formel 1, welches die folgenden Verfahrensstufen aufweist :

a) Umsetzung einer Verbindung der Formel II

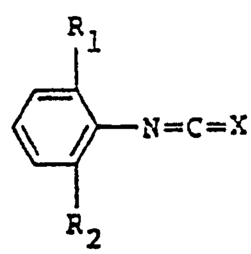
(II),

worin $R_1$, $R_2$ und X die oben angegebenen Bedeutungen haben, mit einer Verbindung der Formel III

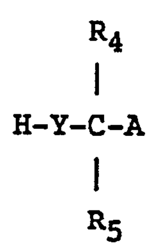
(III),

worin Y, $R_4$ und $R_5$ die oben angegebenen Bedeutungen haben, in einem polaren aprotischen organischen Lösungsmittel bei Raumtemperatur ; und

b) anschliessende Isolierung und Reinigung des Produktes

**Patentansprüche für die Vertragsstaaten GR, ES**

1. Verfahren zur Herstellung einer Verbindung der Formel I

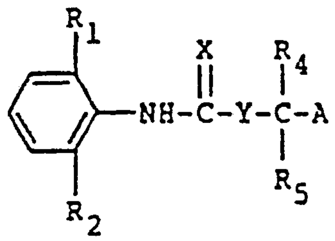

(I),

worin

$R_1$ und $R_2$, die unabhängig voneinander Alkyl mit 1 bis 6 Kohlenstoffatomen, Fluor, Chlor, Brom, $-OR_3$, worin $R_3$ Alkyl mit 1 bis 6 Kohlenstoffatomen oder $-CH_2$-phenyl ;

X Sauerstoff oder Schwefel ;

Y Sauerstoff oder –NH– ;

$R_4$ und $R_5$ unabhängig voneinander Wasserstoff, Alkyl mit 1 bis 6 Kohlenstoffatomen oder zusammen mit dem Kohlenstoffatom, an das sie gebunden sind, einen gesättigten carbocyclischen Ring mit 3 bis 8 Kohlenstoffatomen, der gegebenenfalls mit Alkyl mit 1 bis 4 Rohlenstoffatomen substituiert ist ; und

A eine geradkettige oder verzweigte Kohlenwasserstoffkette mit 7 bis 17 Kohlenstoffatomen, welche gegebenenfalls eine oder zwei Doppelbindungen enthält, bedeuten, welches die folgenden Verfahrensstufen aufweist :

a) Umsetzung einer Verbindung der Formel II

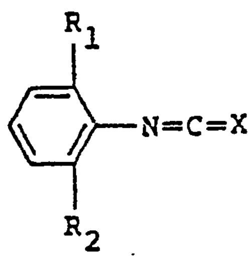

(II),

worin $R_1$, $R_2$ und X die oben angegebenen Bedeutungen haben, mit einer Verbindung der Formel III

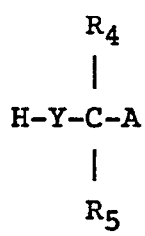

(III),

worin Y, $R_4$ und $R_5$ die oben angegebenen Bedeutungen haben, in einem polaren aprotischen organischen Lösungsmittel bei Raumtemperatur ; und

b) anschliessende Isolierung und Reinigung des Produktes aus Verfahrensstufe (a).

2. Verfahren zur Herstellung einer Verbindung wie in Anspruch 1 definiert, worin X Sauerstoff bedeutet.

3. Verfahren zur Herstellung einer Verbindung wie in Anspruch 1 definiert, worin X Schwefel bedeutet.

4. Verfahren zur Herstellung einer Verbindung wie in Anspruch 1 definiert, worin Y –NH– bedeutet.

5. Verfahren zur Herstellung einer Verbindung wie in Anspruch 1 definiert, ausgewählt aus der aus den nachfolgenden Verbindungen bestehenden Gruppe

N-[2-Ethyl-6-(1-methylethyl)phenyl]-N'-tetradecylharnstoff ;

(Z)-N-[2-Ethyl-6-(1-methylethyl)phenyl]-N'-9-octadecenylharnstoff ;

(Z)-N-(2,6-Dimethylphenyl)-N'-9-octadecenylthioharnstoff ;

(Z)-N-(2,6-Dimethylphenyl)-N'-9-octadecenylharnstoff ;

(Z)-N-(2-Chlor-6-methylphenyl)-N'-9-octadecenylharnstoff ;

(Z)-N-(2,6-(Dichlorphenyl)-N'-9-octadecenylharnstoff ;

N-(2-Chlor-6-methylphenyl)-N'-tetradecylharnstoff ;

N-(2,6-Diethylphenyl)-N'-tetradecylharnstoff ;
(Z)-N-(2,6-Diethylphenyl)-N'-9-octadecenylharnstoff ;
N-(2-Chlor-6-methylphenyl)-N'-octadecylharnstoff ;
N-[2,6-bis(1-Methylethyl)phenyl]-N'-octadecylharnstoff ;
N-(2,6-Diethylphenyl)-N'-tetradecylthioharnstoff ;
N-[2,6-bis(1-Methylethyl)phenyl]-N'-tetradecylthioharnstoff ;
N-Decyl-N'-(2,6-diethylphenyl)-harnstoff ;
N-[2,6-bis(1-Methylethyl)phenyl]-N'-decylharnstoff ;
N-Decyl-N'-[2-ethyl-6-(1-methylethyl)phenyl]-harnstoff ;
N-Decyl-N'-[2-methyl-6-(1-methylethyl)phenyl]-harnstoff ;
N-Decyl-N'-[2-(1,1-dimethylethyl)-6-methylphenyl]-harnstoff ;
N-(2,6-Diethylphenyl)-N'-(1,1-dimethylundecyl)-harnstoff ;
N-[2,6-bis(1-Methylethyl)phenyl]-N'-(1,1-dimethylundecyl)harnstoff ;
N-(1-Decylcyclopentyl)-N'-(2,6-diethylphenyl)harnstoff ;
N-[2,6-bis(1-Methylethyl)phenyl]-N'-(1-decylcyclopentyl)harnstoff.

6. Verfahren zur Verwendung einer nach dem Verfahren nach Anspruch 1 hergestellten Verbindung zur Herstellung einer pharmazeutischen Zubereitung zur Regulierung von Cholesterin.

## Revendications

### P our les Etats Contractants AT, BE, CH, DE, FR, GB, IT, LI, LU, NE, SE

1. Un composé ayant la formule I

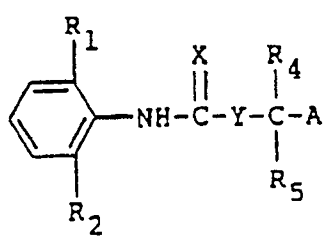

(I),

dans laquelle

$R_1$ et $R_2$, indépendamment l'un de l'autre, sont chacun choisis dans l'ensemble comprenant les radicaux alkyle ayant de un à six atomes de carbone ; le fluor ; le chlore ; le brome ; le radical $OR_3$ où $R_3$ est choisi parmi les radicaux alkyle ayant de un à six atomes de carbone ; ou phénylméthyle ;

X est l'oxygène ou le soufre ;

Y est l'oxygène ou –NH– ;

$R_4$ et $R_5$, chacun indépendamment de l'autre, est choisi parmi l'hydrogène ; les radicaux alkyle ayant de un à six atomes de carbone ; ou encore, quand ils sont réunis à l'atome de carbone auquel ils sont liés, $R_4$ et $R_5$ forment un noyau carbocyclique saturé ayant de trois à huit atomes de carbone, facultativement substitués par un ou plusieurs radicaux alkyle ayant de un à quatre atomes de carbone ; et

A est un radical alkyle à chaîne droite ou ramifiée ayant de six à dix sept atomes de carbone, contenant éventuellement une ou deux doubles liaisons carbone-carbone.

2. Un composé selon la revendication 1, dans lequel X est l'oxygène.

3. Un composé selon la revendication 1, dans lequel X est le soufre.

4. Un composé selon la revendication 1, dans lequel Y est –NH–.

5. Un composé selon la revendication 1, choisi dans l'ensemble comprenant les composés suivants :
N-[2-éthyl-6-(1-méthyléthyl)phényl]-N'-tétradécylurée.
(Z)-N-(2-éthyl-6-(1-méthyléthyl)phényl)-N'-9-octadécénylurée.
(Z)-N-(2,6-diméthylphényl)-N'-9-octadécénylthiourée.
(Z)-N-(2,6-diméthylphényl)-N'-9-octadécénylurée.
(Z)-N-(2-chloro-6-méthylphényl)-N'-9-octadécénylurée.
(Z)-N-2,6-dichlorophényl)-N'-9-octadécénylurée.
N-(2-chloro-6-méthylphényl)-N'-tétradécylurée.

N-(2,6-diéthylphényl)-N'-tétradécylurée.
(Z)-N-(2,6-diéthylphényl)-N'-9-octadécénylurée.
N-(2-chloro-6-méthylphényl)-N'-octadécylurée.
N-[2,6-bis(1-méthyléthyl)phényl]-N'-octadécylurée.
N-(2,6-diéthylphényl)-N'-tétradécylthiourée.
N-[2,6-bis(1-méthyléthyl)phényl]-N'-tétradécylthiourée.
N-Décyl-N'-(2,6-diéthylphényl)urée
N-[2,6-bis(1-méthyléthyl)phényl]-N'-décylurée.
N-Décyl-N'-[2-éthyl-6-(1-méthyléthyl)phényl]urée.
N-Décyl-N'-[2-méthyl-6-(1-méthyléthyl)phényl]urée.
N-Décyl-N'-[2-(1,1-diméthyléthyl)-6-méthylphényl]urée.
N-(2,6-diéthylphényl)-N'-(1,1-diméthylundécyl)urée.
N-[2,6-bis(1-méthyléthyl)phényl]-N'-(1,1-diméthylundécyl)urée.
N-(1-décylcyclopentyl)-N'-(2,6-diéthylphényl)urée.
N-[2,6-bis(1-méthyléthyl)phényl]-N'-(1-décylcyclopentyl)urée.

6. Une composition pharmaceutique contenant un composé selon la revendication 1, en combinaison avec un excipient pharmaceutiquement acceptable.

7. Un procédé de préparation d'une composition pharmaceutique selon la revendication 6, pour réguler le cholestérol.

8. Un procédé de préparation d'un composé de formule 1, comprenant les étapes consistant :

a) à faire réagir un composé de formule II

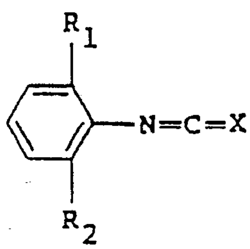 (II),

dans laquelle $R_1$ et $R_2$ et X sont tels que définis ci-dessus, avec un composé de formule III

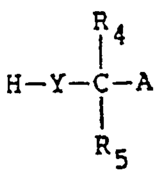 (III),

dans laquelle Y, $R_4$ et $R_5$ sont tels que définis ci-dessus, dans un solvant organique aprotique polaire à la température ambiante ; et

b) à isoler et purifier ensuite le produit de ladite étape de réaction.

**Revendications pour les Etats Contractants GR, ES**

1. Un procédé de préparation d'un composé ayant la formule I

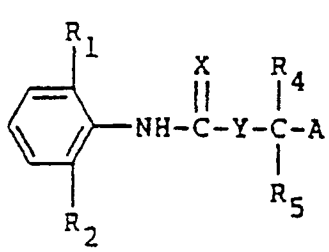 (I),

dans laquelle

$R_1$ et $R_2$, indépendamment l'un de l'autre, sont chacun choisis dans l'ensemble comprenant les radicaux alkyle ayant de un à six atomes de carbone ; le fluor ; le chlore ; le brome ; le radical $OR_3$ où $R_3$ est choisi parmi les radicaux alkyle ayant de un à six atomes de carbone ; ou phénylméthyle ;

X est l'oxygène ou le soufre ;

Y est l'oxygène ou –NH– ;

$R_4$ et $R_5$, chacun indépendamment de l'autre, est choisi parmi l'hydrogène ; les radicaux alkyle ayant de un à six atomes de carbone ; ou encore, quand ils sont réunis à l'atome de carbone auquel ils sont liés, $R_4$ et $R_5$ forment un noyau carbocyclique saturé ayant de trois à huit atomes de carbone, facultativement substitués par un ou plusieurs radicaux alkyle ayant de un à quatre atomes de carbone ; et

A est un radical alkyle à chaîne droite ou ramifiée ayant de six à dix sept atomes de carbone, contenant éventuellement une ou deux doubles liaisons carbone-carbone, comprenant les étapes consistant :

a) à faire réagir un composé de formule II

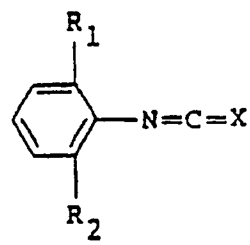

(II),

dans laquelle $R_1$ et $R_2$ et X sont tels que définis ci-dessus, avec un composé de formule 111

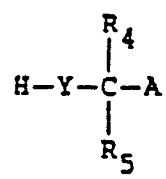

(III),

dans laquelle Y, $R_4$ et $R_5$ sont tels que définis ci-dessus, dans un solvant organique aprotique polaire à la température ambiante ; et

b) à isoler et purifier ensuite le produit de ladite étape de réaction.

2. Un procédé de préparation d'un composé selon la revendication 1, où X est l'oxygène.

3. Un procédé de préparation d'un composé selon la revendication 1, où X est le soufre.

4. Un procédé de préparation d'un composé selon la revendication 1, où Y est –NH–.

5. Un procédé de préparation d'un composé selon la revendication 1, choisi dans l'ensemble comprenant les composés suivants :

N-[2-éthyl-6-(1-méthyléthyl)phényl]-N'-tétradécylurée.
(Z)-N-[2-éthyl-6-(1-méthyléthyl)phényl]-N'-9-octadécénylurée.
(Z)-N-(2,6-diméthylphényl)-N'-9-octadécénylthiourée.
(Z)-N-(2,6-diméthylphényl)-N'-9-octadécénylurée.
(Z)-N-(2-chloro-6-méthylphényl)-N'-9-octadécénylurée.
(Z)-N-(2,6-dichlorophényl)-N'-9-octadécénylurée.
N-(2-chloro-6-méthylphényl)-N'-tétradécylurée.
N-(2,6-diéthylphényl)-N'-tétradécylurée.
(Z)-N-(2,6-diéthylphényl)-N'-9-octadécénylurée.
N-(2-chloro-6-méthylphényl)-N'-octadécylurée.
N-[2,6-bis(1-méthyléthyl)phényl]-N'-octadécylurée.
N-(2,6-diéthylphényl)-N'-tétradécylthiourée.
N-[2,6-bis(1-méthyléthyl)phényl]-N'-tétradécylthiourée.
N-Décyl-N'-(2,6-diéthylphényl)urée.
N-[2,6-bis(1-méthyléthyl)phényl]-N'-décylurée.
N-Décyl-N'-[2-éthyl-6-(1-méthyléthyl)phényl]urée.
N-Décyl-N'-[2-méthyl-6-(1-méthyléthyl) phényl]urée.

N-Décyl-N'-[2-(1,1-diméthyléthyl)-6-méthylphényl]urée.
N-(2,6-diéthylphényl)-N'-(1,1-diméthylundécyl)urée.
N-[2,6-bis(1-méthyléthyl)phényl]-N'-(1,1-diméthylundécyl)urée.
N-(1-décylcyclopentyl)-N'-(2,6-diéthylphényl)urée.
N-[2,6-bis(1-méthyléthyl)phényl]-N'-(1-décylcyclopentyl)urée.

6. Une méthode d'utilisation d'un composé préparé selon la revendication 1 pour préparer une composition pharmaceutique destinée à réguler le cholestérol.